# FASCICULE DE BREVET EUROPEEN

(11) **EP 2 146 994 B1**
(45) Date de publication et mention de la délivrance du brevet: **01.07.2015**
(21) Numéro de dépôt: 08787944.1
(22) Date de dépôt: 11.04.2008
(51) Int. Cl.: C07D 471/18, A61K 31/529, A61P 31/04, C07D 471/08, C07D 211/56, C07D 241/00, A61P 31/00

(54) **COMPOSES HETEROCYCLIQUES AZOTES, LEUR PREPARATION ET LEUR UTILISATION COMME MEDICAMENTS ANTIBACTERIENS**
STICKSTOFFHALTIGE HETEROCYCLISCHE VERBINDUNGEN, IHRE HERSTELLUNG UND IHRE VERWENDUNG ALS ANTIBAKTERIELLE ARZNEIMITTEL
NITROGENOUS HETEROCYCLIC COMPOUNDS, PREPARATION THEREOF AND USE THEREOF AS ANTIBACTERIAL MEDICAMENTS

(30) Priorité: 12.04.2007 FR 0702663
(43) Date de publication de la demande: 27.01.2010
(73) Titulaire: NOVEXEL, 93230 Romainville (FR)
(72) Inventeur: LAMPILAS, Maxime, F-75015 Paris (FR); ROWLANDS, David, Alun, F-78300 Poissy (FR); KEBSI, Adel, F-94320 Thiais (FR); LEDOUSSAL, Benoît, F-77580 Bouleurs (FR); PIERRES, Camille, F-75013 Paris (FR)
(74) Mandataire: Hirsch & Associés
(86) Numéro de dépôt international: PCT/FR2008/000509
(87) Numéro de publication internationale: WO 2008/142285

(56) Documents cités:
- EP-A- 1 798 231
- WO-A-02/10172
- WO-A-02/100860
- WO-A-2004/052891

## Description

L'invention concerne des composés hétérocycliques azotés, leur préparation et leur utilisation comme médicaments antibactériens.

La demande WO 02/100860 décrit les composés répondant à la formule suivante : 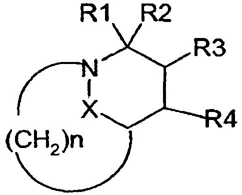 dans laquelle :
a) ou bien R1 représente un atome d'hydrogène, un radical COOH, CN, COOR, (CH₂)n'R₅, CONR₆R₇ ou 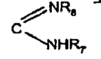
   R est choisi dans le groupe constitué par un radical alkyle renfermant de 1 à 6 atomes de carbone, éventuellement substitué par un radical pyridyle, un radical -CH₂-alkényle renfermant au total de 3 à 9 atomes de carbone, un groupe (poly)alkoxyalkyle renfermant 1 à 4 atomes d'oxygène et 3 à 10 atomes de carbone, un radical aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle renfermant de 1 à 6 atomes de carbone, alkoxy renfermant de 1 à 6 atomes de carbone ou par un ou plusieurs atomes d'halogène,
   R₅ est choisi dans le groupe constitué par un radical COOH, CN, OH, NH₂, CO-NR₆R₇, COOR, OR, OCOH, OCOR, OCOOR, OCONHR, OCONH₂, OSO₂R, NHR, NHCOR, NHCOH, NHSO₂R, NH-COOR, NH-CO-NHR ,NH-CO-NH₂,ou N₃ , R étant défini comme ci-dessus,
   R₆ et R₇, identiques ou différents, sont choisis dans le groupe constitué par un atome d'hydrogène, un radical alkyle renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle,
   n' est égal à 1 ou 2,
   R₃ et R₄ forment ensemble un phényle ou un hétérocycle à caractère aromatique à 5 ou 6 sommets renfermant de 1 à 4 héréroatomes choisis parmi l'azote, l'oxygène et le soufre, et éventuellement substitué par un ou plusieurs groupements R', R' étant choisi dans le groupe constitué par un atome d'hydrogène et les radicaux alkyle renfermant de 1 à 6 atomes de carbone éventuellement substitué par un ou plusieurs radicaux hydroxy, oxo, halogène ou cyano ou par un radical nitro, alkényle renfermant de 2 à 6 atomes de carbone, halogéno, amino, OH, OH protégé, -OR, -NHCOH, -NHCOR, NHCOOR, COOH,
   -COOR, -C(C₆H₅)₃ et -CH₂-CH₂-S(O)m-R, R étant tel que défini précédemment et m étant égal à 0, 1 ou 2,
b) ou bien R₄ représente un atome d'hydrogène ou un groupement (CH₂)_{n'1}R₅, n'1 étant égal à 0, 1 ou 2 et R₅ étant tel que défini ci-dessus,
   et R₁ et R₃ forment ensemble un phényle ou un hétérocycle éventuellement substitué, tel que défini ci-dessus,
   dans les deux cas a) et b)
   R₂ est choisi dans le groupe constitué par un atome d'hydrogène, un atome d'halogène et les radicaux R, S(O)ₘR,
   OR, NHCOR, NHCOOR et NHSO₂R, m et R étant tels que définis précédemment,
   X représente un groupement divalent -C(O)-B- relié à l'atome d'azote par l'atome de carbone,
   B représente un groupement divalent -O-(CH₂)_{n"}- lié au carbonyle par l'atome d'oxygène, un groupement -NR₈-(CH₂)_{n"}- ou -NR_{B}-O-relié au carbonyle par l'atome d'azote, n" est égal à 0 ou 1 et R₈ est choisi dans le groupe constitué par un atome d'hydrogène, un radical OH, R, OR, Y, OY, Y₁, OY₁, Y₂, OY₂, Y₃, O-CH₂-CH₂-S(O)m-R, SiRaRbRc et OSiRaRbRc, Ra, Rb et Rc représentant individuellement un radical alkyle linéaire ou ramifié renfermant de 1 à 6 atomes de carbone ou un radical aryle renfermant de 6 à 10 atomes de carbone, et R et m étant définis comme précédemment.
   Y est choisi dans le groupe constitué par les radicaux COR, COOR, CONH₂, CONHR, CONHOH, CONHSO₂R, CH₂COOH, CH₂COOR, CH₂CONHOH, CH₂CONHCN, CH₂tétrazole, CH₂tétrazole protégé, CH₂SO₃H, CH₂SO₂R, CH₂PO(OR)₂, CH₂PO(OR) (OH), CH₂PO(R) (OH) et CH₂PO(OH)₂.
   Y₁ est choisi dans le groupe constitué par les radicaux SO₂R, SO₂NHCOH, SO₂NHCOR, SO₂NHCOOR, SO₂NHCONHR, SO₂NHCONH₂ et SO₃H,
   Y₂ est choisi dans le groupe constitué par les radicaux PO(OH)₂, PO(OR)₂, PO (OH) (OR) et PO(OH) (R),
   Y₃ est choisi dans le groupe constitué par les radicaux tétrazole, tétrazole substitué par le radical R, squarate,
   NH ou NR tétrazole, NH ou NR tétrazole substitué par le radical R, NHSO₂R et NRSO₂R, R étant défini comme ci-dessus,
   n est égal à 1 ou 2,
ainsi que les sels de ces composés avec des bases ou des acides minéraux ou organiques.

Les atomes de carbone asymétriques contenus dans les composés de formule (I) peuvent indépendamment les uns des autres présenter la configuration R, S ou RS et les composés de formule (I) se présentent donc sous la forme d'énantiomères purs ou de diastéréoisomères purs ou sous la forme d'un mélange d'énantiomères notamment de racémates, ou de mélanges de diastéréoisomères.

En outre, les substituants R₁, R₂, ou R₄ pris individuellement d'une part et X d'autre part pouvant être en position cis et/ou trans par rapport au cycle sur lequel ils sont fixés, les composés de formule (I) se présentent sous la forme d'isomères cis ou d'isomères trans ou de mélanges.

Par ailleurs, la demande WO 04/052891 décrit des composés apparentés.

La demanderesse a découvert que parmi les composés décrits dans la demande WO 02/100860, certains composés particuliers, dont aucun n'est décrit dans la partie expérimentale de ces demandes, possèdent des propriétés antibactériennes tout à fait inattendues.

Le caractère unique des composés de l'invention réside dans le fait qu'ils présentent une excellente activité sur *Pseudomonas aeruginosa,* une souche bactérienne fréquemment rencontrée dans les infections nocosomiales ainsi que chez les patients souffrant de mucoviscidose.

Cette activité intéressante et inattendue n'est pas présente dans les composés préparés dans la demande WO 02/100860 comportant d'autres groupes R₁ que ceux des composés de l'invention. Elle est illustrée plus loin dans la partie expérimentale.

Par ailleurs, les composés de l'invention se sont montrés actifs sur des modèles d'infection animale, y compris sur des souches habituellement résistantes aux antibiotiques communément utilisés. Les composés de l'invention sont capables de contrecarrer les principaux mécanismes de résistance des bactéries que sont les β-lactamases, les pompes à efflux et les mutations des porines.

Les composés de l'invention sont des composés répondant à la formule ci-dessus, dans laquelle R₂ représente un atome d'hydrogène, R₃ et R₄ forment ensemble un hétérocycle azoté à caractère aromatique à 5 sommets, X représente un groupement divalent -C(O)-NRₐ- dans lequel R₈ est un radical -OY₁, Y₁ étant un radical -SO₃H, et surtout :
R₁ représente un radical alkyle substitué par un radical amino ou alkylamino.

L'invention a ainsi pour objet les composés de formule générale (I) 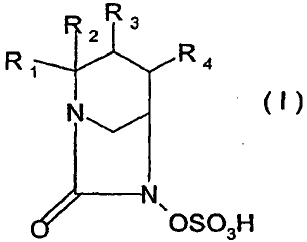 dans laquelle R₁ représente un radical (CH₂)ₙ-NH₂ ou (CH₂)ₙ-NHR, R étant un alkyle (C₁-C₆) et n étant égal à 1 ou 2 ;
R₂ représente un atome d'hydrogène ;
R₃ et R₄ forment ensemble un hétérocycle azoté à caractère aromatique à 5 sommets renfermant 1, 2 ou 3 atomes d'azote éventuellement substitué par un ou plusieurs groupements R', R' étant choisi dans le groupe constitué par un atome d'hydrogène et les radicaux alkyle renfermant de 1 à 6 atomes de carbone ;
sous forme libre, de zwitterions, et sous forme de sels avec les bases et les acides minéraux ou organiques pharmaceutiquement acceptables.

Par radical alkyle renfermant de 1 à 6 atomes de carbone, on entend notamment le radical méthyle, éthyle, propyle, isopropyle, ainsi que butyle, pentyle ou hexyle linéaire ou ramifié.

Par radical alkényle renfermant de 2 à 6 atomes de carbone, on entend notamment le radical allyl et les radicaux butényle, pentényle et hexényle linéaires ou ramifiés.

Par hétérocycle à caractère aromatique, on entend notamment ceux choisis dans la liste qui suit, les deux liaisons symbolisant la jonction avec le cycle azoté (R₃R₄) :

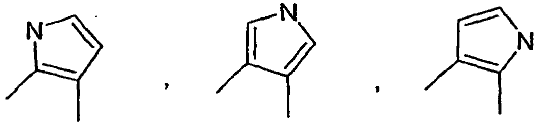

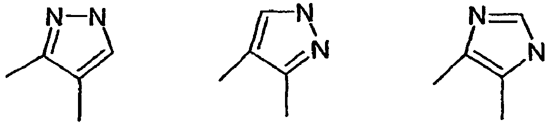

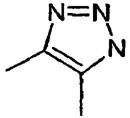

Parmi les sels d'acides des produits de formule (I), on peut citer entre autres, ceux formés avec les acides minéraux, tels que les acides chlorhydrique, bromhydrique, iodhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane et éthane sulfoniques, arylsulfoniques tels que les acides benzène et paratoluènesulfoniques.

Parmi les sels de bases des produits de formule (I), on peut citer, entre autres, ceux formés avec les bases minérales telles que, par exemple, l'hydroxyde de sodium, de potassium, de lithium, de calcium, de magnésium ou d'ammonium ou avec les bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine, ou encore les sels de phosphonium, tels que les alkyl-phosphonium, les aryl-phosphoniums, les alkyl-aryl-phosphonium, les alkényl-aryl-phosphonium ou les sels d'ammoniums quaternaires tels que le sel de tétra-n-butyl-ammonium.

Parmi les composés de formule (I), l'invention a notamment pour objet les composés dans lesquels R₃ et R₄ forment ensemble un hétérocycle pyrazolyle ou triazolyle, éventuellement substitué.

Parmi les composés de formule (I), l'invention a notamment pour objet ceux dans lesquels R1 est choisi dans le groupe constitué par les groupements (CH₂)ₙ-NH₂ et (CH₂)ₙ-NHCH₃, n étant tel que défini précédemment, l'hétérocycle formé par R₃ et R₄ est substitué par un radical alkyle (C₁-C₆).

Parmi les composés de formule (I), l'invention a plus particulièrement pour objet les composés dans lesquels R₁ représente un radical (CH₂)ₙ-NH₂ ou (CH₂)ₙ-NHCH₃, n étant tel que défini précédemment et R₃ et R₄ forment ensemble un cycle pyrazolyle substitué par un radical alkyle (C₁-C₆).

Parmi les composés de formule (I), l'invention a tout particulièrement pour objet :
- la trans 8-(aminométhyl)-4,8-dihydro-1-méthyl-6-oxo-5-(sulfooxy)- 4,7-méthano-7H-pyrazolo[3,4-e] [1,3]diazépin-6(5H)-one,
- la trans 8-(aminométhyl)-4,8-dihydro-6-oxo-5-(sulfooxy)-4,7-méthano-7H-pyrazolo[3,4-e] [1, 3]diazépin-6 (5H)-one,
- la trans 8-(méthylaminométhyl)-4,8-dihydro-6-oxo-5-(sulfooxy)- 4,7-méthano-7H-pyrazolo[3,4-e] [1,3]diazépin-6(5H)-one,
sous forme libre, de zwittterion et de sels avec les bases et les acides minéraux ou organiques pharmaceutiquement acceptables

Un autre objet de l'invention est un procédé permettant la préparation des composés de formule (I).

Ce procédé se caractérise en ce qu'il comporte :
a) une étape au cours de laquelle on fait réagir, avec un agent de carbonylation, le cas échéant en présence d'une base, un composé de formule (II) : 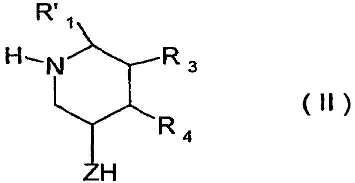 dans laquelle :
   R'₁ représente un radical CN, COOH protégé, COOR ou (CH₂)ₙR'₅,
   R'₅ est un radical OH protégé, CN NH₂ ou NHR protégé, CO₂H protégé, CO₂R
   n, R, R₃ et R₄ sont tels que définis ci-dessus, le substituant aminoalkyle éventuellement présent sur l'hétérocycle formé par R₃ et R₄ étant alors le cas échéant protégé,
   ZH représente un groupement -NHOH protégé, en vue d'obtenir un composé intermédiaire de formule (III) : 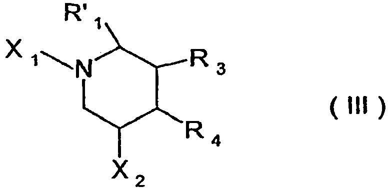 dans laquelle :
      R'₁, R₃ et R₄ ont les mêmes significations que ci-dessus et soit X₁ est un atome d'hydrogène ou un groupe protecteur et X2 représente un groupement -Z-CO-X₃, X₃ représentant le reste de l'agent de carbonylation, soit X₂ est un groupement -ZH et X1 représente un groupement CO-X₃, X₃ étant défini comme ci-dessus ;
b) une étape au cours de laquelle on cyclise l'intermédiaire obtenu précédemment, en présence d'une base ;
   et en ce que :
c) le cas échéant, l'étape a) est précédée et/ou l'étape b) est suivie de l'une ou de plusieurs des réactions suivantes, dans un ordre approprié :
   - protection des fonctions réactives,
   - déprotection des fonctions réactives,
   - estérification
   - saponification,
   - sulfatation,
   - réduction d'esters,
   - alkylation,
   - carbamoylation,
   - formation d'un groupe azido,
   - réduction d'un azido en amine,
   - salification,
   - échange d'ions,
   - dédoublement ou séparation de diastéréoisomères.

Comme agent de carbonylation, on peut mettre en oeuvre un réactif tel que le phosgène, le diphosgène, le triphosgène, un chloroformiate d'aryle tel que le chloroformiate de phényle ou de p-nitrophényle, un chloroformiate d'aralkyle tel que chloroformiate de benzyle, un chloroformiate d'alkyle ou d'alkényle tel que le chloroformiate de méthyle ou d'allyle, un dicarbonate d'alkyle tel que le dicarbonate de tert-butyle, le carbonyl-diimidazole et leurs mélanges, le disphosgène étant préféré.

La réaction a lieu de préférence en présence d'une base ou d'un mélange de bases qui neutralise l'acide formé. Elle peut notamment être une amine telle que la triethylamine, la diisopropyléthylamine, la pyridine, la diméthylaminopyridine. Toutefois, on peut également opérer en utilisant le produit de départ de formule II comme base. On en utilise alors un excès.

Le cas échéant, le produit de formule II est mis en oeuvre sous la forme d'un sel d'acide, par exemple un chlorhydrate ou un trifluoroacétate.

Comme base dans l'étape b), on peut également utiliser les amines, ou encore les hydrures, les alcoolates, les amidures ou carbonates de métaux alcalins ou alcalino-terreux.

Les amines peuvent être choisies par exemple dans la liste ci-dessus.

Comme hydrure on peut notamment utiliser l'hydrure de sodium ou de potassium.

Comme alcoolate de métal alcalin, on utilise de préférence le t-butylate de potassium.

Comme amidure de métal alcalin on peut notamment utiliser le bis(triméthylsilyl) amidure de lithium.

Comme carbonate, on peut notamment utiliser le carbonate ou bicarbonate de sodium ou de potassium.

Le cas échéant, l'intermédiaire de formule III peut être obtenu sous forme d'un sel d'acide généré lors de la réaction de carbonylation et notamment un chlorhydrate. Il est ensuite mis en oeuvre dans la réaction de cyclisation sous cette forme.

De préférence, la cyclisation est effectuée sans isolement de l'intermédiaire de formule III.

Les réactions mentionnées à l'étape c) sont d'une manière générale des réactions classiques, bien connues de l'homme du métier. Des exemples de conditions utilisées sont décrits dans la demande WO 02/100860 ou encore dans la demande 04/052891.

Les fonctions réactives qu'il convient, le cas échéant, de protéger sont les fonctions acides carboxyliques, amines, amides, hydroxy et hydroxylamines.

La protection de la fonction acide est notamment effectuée sous forme d'esters d'alkyle, d'esters allyliques, de benzyle, benzhydryle ou p-nitrobenzyle.

La déprotection est effectuée par saponification, hydrolyse acide, hydrogénolyse, ou encore clivage à l'aide de complexes solubles du Palladium O.

Des exemples de ces protections et déprotections sont fournis dans la demande WO 02/100860.

La protection des amines, des azotes hétérocycliques et des amides est notamment effectuée, selon les cas, sous forme de dérivés benzylés ou tritylés, sous forme de carbamates, notamment d'allyle, benzyle, phényle ou tertbutyle, ou encore sous forme de dérivés silylés tels que les dérivés tertbutyle diméthyl, triméthyl, triphényl ou encore diphényl tertbutylsilyle, ou de dérivés phénylsulfonylalkyle ou cyanoalkyle.

La déprotection est effectuée, selon la nature du groupement protecteur, par le sodium ou le lithium dans l'ammoniac liquide, par hydrogénolyse ou à l'aide de complexes solubles du Palladium O, par action d'un acide, ou par action du fluorure de tétrabutylammonium ou de bases fortes telles que l'hydrure de sodium ou le t.butylate de potassium.

La protection des hydroxylamines est effectuée notamment sous forme d'éthers de benzyle ou d'allyle.

Le clivage des éthers est effectué par hydrogénolyse ou à l'aide de complexes solubles du Palladium O.

La protection des alcools et des phénols est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent être par exemple des carbonates de méthyle, tertbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium.

Des exemples sont fournis dans la partie expérimentale.

La réaction de sulfatation est effectuée par action des complexes SO₃-amines tels que SO₃-pyridine ou SO₃-diméthylformamide, en opérant dans la pyridine, le sel formé, par exemple le sel de pyridine, pouvant ensuite être échangé par exemple par un sel d'une autre amine, d'un ammonium quaternaire ou d'un métal alcalin. Un exemple est fourni dans la partie expérimentale.

La réaction d'alkylation est effectuée par action sur les dérivés hydroxylés, les énolates d'esters ou de cétones, les amines ou les azotes hétérocycliques, selon les cas, d'un sulfate d'alkyle ou d'un halogènure d'alkyle ou d'alkyle substitué, notamment par un radical carboxy libre ou estérifié. Des réactions d'alkylation peuvent également être réalisées par amination réductrice.

La salification par les acides est le cas échéant réalisée par addition d'un acide en phase soluble au composé. La salification par les bases de la fonction sulfooxy peut être réalisée à partir du sel de pyridinium obtenu lors de l'action du complexe SO3-pyridine et on obtient les autres sels à partir de ce sel de pyridinium. On peut encore opérer par échange d'ions sur résine.

La réaction de carbamoylation peut être réalisée par la mis en oeuvre d'un chloroformiate ou d'un réactif type Boc-ON puis d'une amine ou, le cas échéant, d'ammoniac.

L'introduction d'un groupe azido peut être effectuée par exemple par action d'azoture de sodium sur un intermédiaire de type mésylate ou par des réactions de type Mitsunobu.

La réduction d'un groupe azide peut être effectuée par action de trialkyl ou triarylphosphine.

La séparation des énantiomères et diastéréoisomères peut être réalisée selon les techniques connues de l'homme du métier, notamment la chromatographie.

Outre via les procédés décrits précédemment, des composés de formule (I) peuvent être obtenus par des méthodes qui utilisent au départ un composé de formule (II) dans laquelle R'₁, R₃, R₄ et HZ ont les valeurs qui conduisent directement (sans transformation) à celles des composés que l'on souhaite préparer. Le cas échéant, celles de ces valeurs qui renfermeraient des fonctions réactives telles que mentionnées plus haut sont alors protégées, la déprotection intervenant à l'issue de l'étape de cyclisation b ou à tout autre moment opportun dans la synthèse. Les protections et déprotections sont alors réalisées comme décrit ci-dessus.

L'invention a encore pour objet un procédé selon ce qui précède, caractérisé en ce que le composé de formule (II) est obtenu par un procédé selon lequel on traite un composé de formule (IV) : 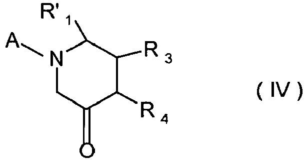 dans laquelle R'₁, R₃ et R₄ sont définis comme précédemment, et A représente un atome d'hydrogène ou un groupement protecteur de l'azote, par un agent de réduction, pour obtenir un composé de formule (V) : 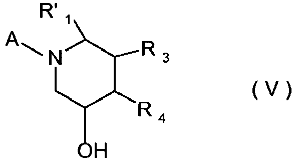 dans laquelle A, R'₁, R₃ et R₄ conservent leur signification précitée, dans lequel, le cas échéant, l'on remplace le groupement OH par un groupe partant, pour obtenir un composé de formule (VI) : 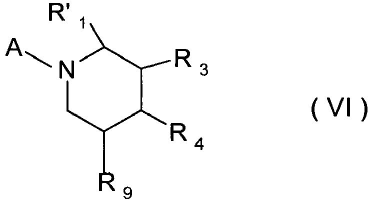 dans laquelle A, R'₁, R₃ et R₄ conservent leur signification précitée et R9 représente un groupe partant, que l'on traite par un composé de formule Z₁H₂ dans laquelle Z₁ représente un groupement -HN-OH protégé puis, le cas échéant, par un agent de déprotection de l'atome d'azote approprié.

L'invention a encore pour objet un procédé selon ce qui précède, caractérisé en ce que le composé de formule (II) est obtenu par un procédé selon lequel on traite un composé de formule (IV) telle que définie précédemment, par l'hydroxylamine protégée au niveau de l'hydroxy, pour obtenir un composé de formule (VII) : 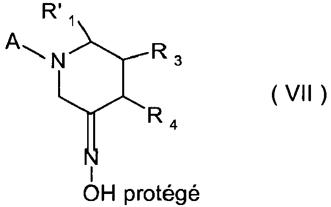 dans laquelle A, R'₁, R'₂, R₃, R'₄, n et R'₈ sont définis comme précédemment, que l'on fait réagir avec un agent de réduction pour obtenir un composé de formule (VIII) : 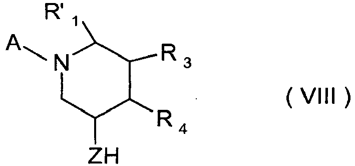 dans laquelle A, R'₁, R₃, R₄, n" et ZH sont définis comme précédemment, que l'on traite, le cas échéant, par un agent de déprotection de l'atome d'azote approprié.

Le groupement protecteur de l'azote est notamment l'un de ceux qui sont cités plus haut.

L'agent de réduction est notamment un borohydrure alcalin.

Le groupe partant est notamment un sulfonate, par exemple un mésylate ou un tosylate, obtenu par action de chlorure de sulfonyle correspondant en présence d'une base, ou un halogène, plus particulièrement un chlore, un brome ou un iode, obtenu par exemple par action du chlorure de thionyle ou de P(C₆H₅)₃CBr₄ ou PBr₃ ou, dans le cas d'un atome d'iode, par action d'un iodure alcalin sur un sulfonate.

L'agent de déprotection est notamment l'un de ceux mentionnés plus haut.

L'agent de réduction que l'on fait agir sur le composé de formule (VII) est notamment un cyano ou un acétoxyborohydrure de sodium.

Comme indiqué plus haut, les composés de formule générale (I) possèdent une excellente activité antibiotique sur *Pseudomonas aeruginosa* ainsi que sur des modèles d'infection animale par des souches résistantes aux agents anti-bactériens communément utilisés. Cette activité antibiotique remarquable et inattendue n'avait pas été observée pour les composés décrits dans la demande WO 02/100860.

Ces propriétés rendent aptes lesdits composés, sous forme libre, de zwitterions ou de sels d'acides et de bases pharmaceutiquement acceptables à être utilisés comme médicaments dans le traitement des infections sévères à *Pseudomonas,* notamment les infections nosocomiales et, d'une manière générale, les infections majeures chez les sujets à risques. Il peut en particulier s'agir d'infections des voies respiratoires, par exemple la pneumonie aiguë ou les infections chroniques des voies inférieures, les infections du sang, par exemple les septicémies, les infections aiguës ou chroniques des voies urinaires, celles du système auditif, par exemple l'otite externe maligne, ou l'otite chronique suppurante, celles de la peau et des tissus mous, par exemple les dermatites, les plaies infectées, la folliculite, la pyodermite, les formes rebelles d'acnée, les infections des yeux, par exemple l'ulcère de la cornée, celles du système nerveux, notamment les méningites et les abcès du cerveau, les infections cardiaques telles que l'endocardite, les infections des os et des articulations telles que la pyoarthrose sténoarticulaire, l'ostéomyélite vertébrale, la symphysite pubienne, les infections du tube gastro-intestinal, telles que l'entérocolite nécrosante et les infections péri-rectales.

La présente invention a donc également pour objet, à titre de médicaments et notamment de médicaments antibiotiques, les composés de formule (I) tels que définis ci-dessus.

Parmi les composés de formule (I), l'invention a notamment pour objet à titre de médicaments les composés dans lesquels R₃ et R₄ forment ensemble un hétérocycle pyrazolyle ou triazolyle, éventuellement substitué, et parmi ceux-ci, ceux dans lesquels R₁ est choisi dans le groupe constitué par les groupements (CH₂)ₙ-NH₂ et (CH₂)ₙ-NHCH₃, n étant tel que défini précédemment, l'hétérocycle formé par R₃ et R₄ est substitué par un radical alkyle (C₁-C₆).

Parmi les composés de formule (I), l'invention a plus particulièrement pour objet à titre de médicaments, les composés dans lesquels R₁ représente un radical (CH₂)ₙ-NH₂ ou (CH₂)ₙ-NHCH₃, n étant tel que défini précédemment et R₃ et R₄ forment ensemble un cycle pyrazolyle substitué par un radical alkyle (C₁-C₆).

Parmi les composés de formule (I), l'invention a tout particulièrement pour objet à titre de médicament,
- la trans 8-(aminométhyl)-4,8-dihydro-1-méthyl-6-oxo-5-(sulfooxy)- 4,7-méthano-7H-pyrazolo[3,4-e] [1,3]diazépin-6(5H)-one,
- la trans 8-(aminométhyl)-4,8-dihydro-6-oxo-5-(sulfooxy)-4,7-méthano-7H-pyrazolo[3,4-e] [1,3]diazépin-6(5H)-one,
- la trans 8-(méthylaminométhyl)-4,8-dihydro-6-oxo-5-(sulfooxy)- 4,7-méthano-7H-pyrazolo[3,4-e][1,3]diazépin-6(5H)-one,
sous forme libre, de zwittterion et de sels avec les bases et les acides minéraux ou organiques pharmaceutiquement acceptables.

L'invention a aussi pour objet les compositions pharmaceutiques renfermant comme principe actif, au moins un des composés selon l'invention tels que définis ci-dessus.
Ces compositions peuvent être administrées par voie buccale, rectale, parentérale, notamment intramusculaire, ou par voie locale en application topique sur la peau et les muqueuses.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'un lyophilisat destiné à être dissout extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 10 g par jour, par voie orale chez l'homme, avec le produit décrit à l'exemple 1 ou encore comprise entre 0,25 g et 10 g par jour par voie intramusculaire ou intraveineuse.

Les produits de formule (I) peuvent également être utilisés comme désinfectants des instruments chirurgicaux.

Les exemples suivants illustrent l'invention.

### EXEMPLES

### Exemple 1 sel de sodium et de trifluoroacétate de la trans 8-(aminométhyl)-4,8-dihydro-1-méthyl-6-oxo-5-(sulfooxy)-4,7-méthano-7H-pyrazolo[3,4-e] [1,3] diazépin-6(5H)-one

### Stade A :

### 4,7-dihydro-1-méthyl-4-((phénylméthoxy)amino)-1H-pyrazolo[3,4-c]pyridine-6(5H),7-dicarboxylate de 6-(1,1-diméthyléthyle) et de 7-méthyle (B)

Le dérivé **A** (4,7-dihydro-4-hydroxy-1-methyl-1H-pyrazolo[3,4-*c*]pyridine-6 (5*H*),7-dicarboxylate de 6-(1,1-diméthyléthyle) et de 7-méthyle, décrit dans la demande WO 02100860 (10 g, 32.12 mmol) est mis en suspension dans le dichlorométhane (100 ml) à température ambiante sous azote et sous agitation. La suspension se dissout après l'ajout de triethylamine (14.30 ml, 10.28 mmol, 3.2eq). Au milieu réactionnel refroidi à -78°C est additionné goutte à goutte une solution de chlorure de méthane sulfonyle (11.4 ml, 96.36 mmol, 3eq) dans le dichlorométhane (12 ml, 1 volume). Après 30 min de contact, l'alcool A est complètement transformé en mésylate.

Une solution de O-benzyl-hydroxylamine dans le dichlorométhane est fraîchement préparée à partir du chlorhydrate de O-benzylhydroxylamine (25.4 g, 160.6 mmol, 5eq). Le chlorhydrate de O-benzylhydroxylamine est dissout dans un mélange de dichlorométhane (100 ml) et d'eau (50 ml). Une solution de soude 2N (85 ml, 176.66 mmol) est ajoutée à 0°C. Après 10 min de contact et décantation, la phase organique est séchée sur sulfate de magnésium durant 45 min, puis concentrée à demi volume. L'addition de cette solution au mésylate préparé ci-dessus se fait à -78°C goutte à goutte sur 1 heure. Le mélange réactionnel est agité en laissant la température remonter progressivement à l'ambiante. On traite par addition d'eau (200 ml) et dilue le milieu avec du dichlorométhane (100 ml), agite, décante puis réextrait la phase aqueuse au dichlorométhane. La phase organique est lavée avec une solution de NaCl saturée (200 ml), séchée, puis concentrée à sec. On récupère une poudre amorphe blanche , qui après chromatographie délivre le dérivé **B** attendu (8.25 g, 66%).
MS (ES (+)) : m/z [M⁺] = 417.2
¹H NMR (400MHz, CDCl3): un diastéréoisomère (2 rotamères) δ (ppm) = 1.43 (s, 9H, tBu), 3.15 (dd, 1H, N-CH2-CH-N), 3.68 / 3.70 (s, 3H, CH3), 3.84(s, 3H, CH3), 3.98 (m, 2H, N-CH2-CH-N), 4.6-4.8 (massif, 3H, NH-O-CH2-Ph et N-CH2-CH-N), 5.40 / 5.8 (s, 1H, CH-CO2Me), 7.22-7.31(massif, 5H, Ph), 7.40(s, 1H, H pyrazole)

### Stade B :

### Trans 1-methyl-6-oxo-5-(phénylméthoxy)4,5,6,8-tétrahydro-4,7-méthano-1H-pyrazolo[3,4-e] [1,3]diazépine-8 (7H) carboxylate de méthyle (C)

Une solution 4N de HCl/ dioxane (400 ml,15eq) est coulée sur une solution de **B** (21 g, 50.42 mmol) dissout dans le dioxane (50 ml) à température ambiante. Le mélange réactionnel est agité pendant 30 min, puis le dioxane est évaporé. Le résidu est repris sous agitation dans un mélange d'eau (100 ml) et d'acétate d'éthyle (500 ml). Une solution d'ammoniaque concentrée à 20% (42 ml) est ajoutée à 0°C. L'agitation est poursuivie pendant 30 min. Après décantation la phase aqueuse est ré-extraite avec de l'acetate d'éthyle (2*300 ml), la dernière extraction étant réalisée après saturation de la phase aqueuse avec du NaCl. La phase organique est séchée puis concentrée. On obtient l'intermédiaire pipéridine déprotégée sous forme d'une huile jaune (m=15.7 g, 98%) qui est repris dans l'acétonitrile (400 ml). A ce mélange refroidi à 0°C, sont ajoutés la triéthylamine (21 ml, 151.2 mmol, 3eq), puis le diphosgène (3.04 ml, 25.2 mmol, 0.5eq) coulé goutte à goutte sur 30 min. Après une nuit de contact à température ambiante, le milieu est concentré puis repris avec de l'acétate d'éthyle (500 ml) et traité avec une solution d'acide tartrique 10% (200 ml). Le mélange est agité, décanté. La phase organique est lavée par une solution d'acide tartrique 10% (2*200 ml), par une solution de NaCl saturée, puis séchée et concentrée sous pression réduite. Le produit blanc obtenu (m=15.3 g, 89%) est repris dans le dichlorométhane (150 ml). Du 1-8-Diazabicyclo[5.4.0]undéc-7-ène (7.53 ml,50.04 mmol) est ajouté goutte à goutte. Le mélange est agité pendant 2h, traité avec de l'eau (200 ml), agité, décanté. La phase organique est lavée avec de l'eau (2*200 ml), puis avec une solution de NaCl saturée (1*200 ml), et séchée sur MgSO₄, puis concentrée à sec.

On récupère le dérivé **C** attendu (m=14.72 g, 85%), sous forme d'un solide blanc
MS (ES (+)) : m/z [M⁺]=343
¹H NMR (400MHz, CDCl3): δ (ppm) = 3.25 (d, 1H, N-CH2-CH-N), 3.45 (d, 1H, N-CH2-CH-N), 3.80 (s, 3H, CH3), 3.88 (s, 3H, CH3), 3.9 (s,1H, N-CH2-CH-N), 4.7 (d, 1H, N-O-CH2-Ph), 5.02 (d, 1H, N-O-CH2-Ph), 5.22 (s, 1H, CH-CO2Me), 7.39-7.43(massif, 6H, H pyrazole + Ph)

### Stade C :

### 4,8-dihydro-8-(hydroxyméthyl)-1-méthyl-5-(phénylméthoxy)-4,7-méthano-7H-pyrazolo[3,4-e] [1,3] diazépin-6(5H)-one (D)

Une solution de C (5g, 14.60 mmol) dans un mélange de tétrahydrofurane (150 ml) / méthanol (50 ml) anhydres, sous azote et sous agitation, est refroidie à -10°C. Du borohydrure de lithium (668 mg, 30.67 mmol, 1.2eq) est ajouté au milieu réactionnel. Après 2 h d'agitation à -10°C, 1.2eq supplémentaires de LiBH4 sont ajoutés. La réaction est traitée à froid 2 h plus tard par une solution à 10% de NaH2PO4. Le tétrahydrofurane et le méthanol sont évaporés sous pression réduite(200 mbar, 40°C). Le mélange résiduel est repris avec de l'acétate d'éthyle (200 ml), agité et décanté. La phase aqueuse est réextraite avec de l'acétate d'éthyle (100 ml). La phase organique est séchée sur sulfate de magnésium puis concentrée à sec. La poudre jaune clair obtenue (6.6 g) est chromatographiée sur silice (éluant-acétate d'éthyle) pour donner le dérivé **D** (3.2 g,10.18 mmol, 64%).
MS (ES (+)) : m/z [M⁺]=315
¹H NMR (400 MHz, DMSO-_{d6}): δ (ppm) = 3.16 (dd, 1H, N-CH2-CH-N), 3.48 (d, 1H, N-CH2-CH-N), 3.71 (s, 3H, CH3), 3.81-3.91 (massif, 2H, CH2OH), 4.44 (m, 1H, N-CH2-CH-N), 4.48 (m, 1H, CHCH2OH), 4.88 (m, 2H, N-O-CH2-Ph), 5.20 (m, 1H, OH), 7.35-7.40 (massif, 6H, H pyrazole + Ph).

### Stade D :

### Trans 4,8-dihydro-1-méthyl-8-[(méthylsulfonyl)oxyméthyl)]- 5-(phénylméthoxy)-4,7-méthano-7H-pyrazolo[3,4-e] [1,3] diazépin-6(5H)-one (E)

Le dérivé **D** (2.76 g, 8.78 mmol) est mis en solution dans du dichloromethane (100 ml) à température ambiante sous azote et sous agitation. Après refroidissement à 0°C, on ajoute de la triethylamine (1.83 ml, 13.17 mmol, 1.5eq), puis goutte à goutte une solution de chlorure de mesyle (1.61 g, 14.05 mmol) dans le dichloromethane (100 ml). Le bain de glace est retiré en fin d'addition. Au bout d'une heure de contact à température ambiante, la réaction est traitée sous agitation par une solution à 10% de NaH2PO4 (80 ml). Après agitation et décantation, la phase aqueuse est réextraite au dichlorométhane (50 ml). La phase organique est séchée, puis concentrée sous pression réduite pour donner le dérivé attendu (3.44 g, rendement quantitatif).
MS (ES (+)) : m/z [M⁺]=393
¹H NMR (400 MHz, DMSO-d₆): δ (ppm) = 3.23 (dd, 1H, N-CH2-CH-N), 3.26 (s, 3H, CH3), 3.45 (d,1H, N-CH2-CH-N), 3.76(s, 3H, CH3), 4.52 (m, 1H, N-CH2-CH-N), 4.58 (dd, 1H, CH-CH2-OMs), 4.66 (dd, 1H, CH-CH2-OMs), 4.88 (m, 3H, CHCH2OMs et N-O-CH2-Ph), 7.35-7.45 (massif, 6H, H pyrazole + Ph)

### Stade E :

### trans 8-(azidométhyl)-4,8-dihydro-1-méthyl-5-(phénylméthoxy)-4,7-méthano-7H-pyrazolo[3,4-e] [1,3] diazépin-6(5H)-one (F)

De l'azidure de sodium est ajouté en une seule fois (1.71 g, 26.3 mmol) à une solution de **E** (3.44 g, 8.78 mmol) dans le dimethylformamide (70 ml) à température ambiante sous azote et sous agitation. Le milieu réactionnel est chauffé à 65°C toute une nuit, puis traité par une solution aqueuse à 10% de NaH2PO4 (50 ml). Après agitation et décantation, la phase aqueuse est réextraite au dichloromethane (2*50 ml). La phase organique est séchée, puis concentrée sous pression réduite pour donner 3.96 g de dérivé **F** attendu (3 g, 8.78 mmol).
MS (ES (+)) : m/z [M⁺]=340
¹H NMR (400 MHz, DMSO-*d*₆): δ (ppm) =3.20 (dd, 1H, N-CH2-CH-N), 3.48 (d, 1H, N-CH2-CH-N), 3.66 (dd, 1H, CH-CH2-N3), 3.72 (s, 3H, CH3), 3.92 (dd, 1H, CH-CH2-N3), 4.50(d, 1H, N-CH2-CH-N), 4.76 (dd, 1H, CHCH2ON3), 4.89 (m, 2H, N-O-CH2-Ph), 7.35-7.45 (massif, 6H, H pyrazole + Ph)

### Stade F :

### trans [[4,8-dihydro-1-méthyl-6-oxo-5-(phénylméthoxy)-4,7-méthano-7H-pyrazolo[3,4-e] [1,3] diazépin-8-yl]méthyl]-carbamate de 1,1-diméthyléthyle (G)

Une solution molaire de trimethylphosphine (3.4 ml, 3.4 mmol) est ajoutée goutte à goutte à une solution de **F** (1.15 g, 3.39 mmol) dans un mélange toluène (5 ml) et tetrahydrofurane (5 ml) à température ambiante sous azote et sous agitation. Apres 3 H de contact, une solution de BOC-ON (0.92 g, 3.6 mmol) dans le tetrahydrofurane (10 ml) est ajoutée goutte à goutte au milieu réactionnel refroidi à 0°C. L'agitation est poursuivie pendant 3 h à température ambiante. Le milieu réactionnel est traité par une solution aqueuse à 10% de NaHCO3 (50 ml). Après agitation et décantation, la phase aqueuse est réextraite à l'acétate d'éthyle (50 ml). La phase organique est séchée, puis concentrée sous pression réduite pour donner une huile (2.2 g). Le produit brut est chromatographié sur colonne de silice (éluant cyclohexane/ acétate d'éthyle 5/5). On obtient le produit attendu (0.62 g, 1.49 mmol, 70%).
MS (ES (+)) : m/z [M⁺]=414
¹H NMR (400MHz, CDCl3): δ (ppm) = 1.39 (s, 9H, tBu), 3.05 (dd, 1H, N-CH2-CH-N), 3.19 (dd, 1H, CH-CH2-NHBOC), 3.27 (dd, 1H, N-CH2-CH-N), 3.72 (s, 3H, CH3), 3.78(m, 1H, CH-CH2-NHBOC), 3.88 (d, 1H, N-CH2-CH-N), 4.48 (dd , 1H, CHCH2NHBOC), 4.79 (d, 1H, N-O-CH2-Ph), 4.92 (d, 1H, N-O-CH2-Ph), 5.18 (m, 1H, H mobile), 7.35 (s, 1H, H pyrazole), 7.37-7.48 (massif, 5H, Ph)

### Stade G :

### Sel de pyridinium du trans [[4,8-dihydro-1-méthyl-6-oxo-5-(sulfooxy)-4,7-méthano-7H-pyrazolo[3,4-e] [1,3] diazépin-8-yl]méthyl]-carbamate de 1,1-diméthyléthyle (H).

Du palladium 10% sur charbon (140 mg) est ajouté à une solution de **G** (0.6 g, 1.45 mmol) dans le méthanol (10 ml). Le milieu réactionnel est hydrogéné pendant 3h. Le méthanol est ensuite évaporé sous pression réduite pour donner l'intermédiaire débenzylé.
MS (ES (+)) : : m/z [M⁺]=324

L'intermédiaire débenzylé est repris dans la pyridine (3 ml) en présence du complexe pyridine / sulfure de trioxyde (462 mg, 2.9 mmol). La réaction est maintenue sous agitation à température ambiante pendant une nuit. Le milieu est ensuite concentré sous pression réduite. Le brut réactionnel est chromatographié sur colonne de silice (éluant dichloromethane 100% puis gradient avec du méthanol de 5% à 20%) pour donner le dérivé **H** (0.49 g, 1.25 mmol, 84%).
MS (ES (+)) : m/z [M⁻]=402
¹H NMR (400 MHz, DMSO-*d*₆) : δ (ppm) =1.41 (s, 9H, tBu), 3.30-3.80 (massif, 4H, 2 CH2), 3.72(s, 3H, CH3), 4.42 (dd, 1H, CHCH2ONHBOC), 4.64 (d, 1H, N-CH2-CH-N), 7.21 (m, 1H, H mobile), 7.35 (s, 1H, H pyrazole), 8.02 (dd, 2H, pyridine), 8.54 (m, 1H, pyridine), 8.91 (m, 2H, pyridine)

### Stade H :

### Sel de sodium de la trans [[4,8-dihydro-1-méthyl-6-oxo-5-(sulfooxy)-4,7-méthano-7H-pyrazolo[3,4-e) [1,3] diazépin-8-yl]méthyl]-carbamate de 1,1-diméthyléthyle (I)

Une suspension de 60 g de résine DOWEX 50WX8 dans une solution de soude 2N (300 ml) est agitée pendant une heure, puis versée sur une colonne à chromatographie. On élue à l'eau déminéralisée jusqu'à pH neutre, puis conditionne la colonne avec un mélange eau/THF 90/10. Le dérivé **H** (0.49 g, 1.01 mmol) est dissout dans un minimum d'eau, déposé sur la colonne, puis élué avec un mélange eau/THF 90/10. Les fractions contenant le substrat sont réunies et congelées. La solution congelée est lyophilisée pour conduire au produit **I** attendu (0.44 g, 1.03 mmol, 100%).
MS (ES (-)) : m/z [M⁻]=402
¹H NMR (400 MHz, DMSO-*d*₆): δ (ppm) =1.39 (s, 9H, tBu), 3.30-3.72 (m, 7H, 2 CH2, CH3), 4.42 (m, 1H, CHCH2ONHBOC), 4.64 (s, 1H, N-CH2-CH-N),7.16 (m, 1H, H mobile), 7.35 (s, 1H, H pyrazole).

### Stade I :

### Sel de sodium et de trifluoroacétate de la trans 8-(aminométhyl)-4,8-dihydro-1-méthyl-6-oxo-5-(sulfooxy)-4,7-méthano-7H-pyrazolo[3,4-e] [1,3] diazépin-6(5H)-one (J)

Une solution d'acide trifluoroacétique (10 ml) dans le dichloromethane (10 ml) est coulée goutte à goutte sur une solution de **I** (0.15 g, 0.35 mmol) dans le dichloromethane (5 ml) sous azote et refroidie à 0°C. La réaction est maintenue sous agitation pendant 1h à température ambiante. Le mélange est évaporé à sec et repris dans un minimum d'eau. La solution est congelée puis lyophilisée pour donner le dérivé **J** attendu (193 mg, 0.35 mmol, 100%).
MS (ES (-)) : m/z [M⁻]=301
¹H NMR (400 MHz, DMSO-*d*₆): δ (ppm) =3.32 (dd, 1H, N-CH2-CH-N), 3.33-3.37 (m, 2H, 2CH), 3.43 (d, 1H, N-CH2-CH-N), 3.74 (s, 3H, CH3), 4.73 (m, 2H, CH-CH2-NH3+), 7.41(s, 1H, H pyrazole), 8.10 (m, 3H, NH3⁺)

### Exemple 2 : Le sel de sodium et de trifluoroacétate de la trans 8-(amino-méthyl)-4,8-dihydro-6-oxo-5-(sulfooxy)-4,7-méthano-7H-pyrazolo[3,4-e] [1,3] diazépin-6(5H)-one

### Stade A _{:}

### trans-8-(hydroxyméthyl)-4,5,6,8-tétrahydro-6-oxo-5-(phénylméthoxy)-4,7-méthano-7H-pyrazolo[3,4-e][1,3]diazépin-6(5H)-one

L'ester trans-4,5,6,8-tétrahydro-6-oxo-5-(phénylméthoxy)-4,7-méthano-7*H*-pyrazolo[3,4-e][1,3]diazépine-8-carboxylate de méthyle décrit dans le brevet WO 2004/052891 (Exemple 1, stade K) (5 g, 15.2 mmol) est mis en solution dans un mélange méthanol/tétrahydrofurane anhydres 1/1 (100 mL), sous azote. Du NaBH₄ (2.3 g, 60.9 mmol) est alors ajouté par portions. Après une nuit d'agitation à température ambiante, le mélange réactionnel est traité avec une solution aqueuse 10% de NaH₂PO₄ (100 mL). Après évaporation à sec, le mélange réactionnel est repris dans l'eau. Le précipité formé est agité une nuit dans la glace, puis filtré et séché au moins 24h sous vide en présence de P₂O₅, pour donner le composé attendu (3.30 g, 11.0 mmol, 72%) sous forme de poudre blanche.
MS (ES(+)) : m/z [M+H]⁺ = 301
¹H RMN (400MHz, DMSO-*d*₆): δ(ppm) = 3.18-3.50 (ABX, 2H, N-CH₂-CH-N), 3.65-3.76 (ABX, 2H, N-CH-CH₂-OH), 4.34 (t, 1H, N-CH-CH₂-OH), 4.46 (d, 1H, N-CH₂-CH-N) , 4.88 (s, 2H, CH₂-Ph), 7.29-7.43 (m, 5H, Ph), 7.66 (s, 1H, H pyrazole), 12.72 (broad, 1H, OH).

### Stade B :

### trans [[4,8-dihydro-6-oxo-5-(phénylméthoxy)-4,7-méthano-7H-pyrazolo[3,4-e] [1,3] diazépin-8-yl]méthyl]-carbamate de 1,1-diméthyle

L'alcool obtenu au stade A de l'exemple 2 (1.73 g, 5.76 mmol) est mis en solution dans la pyridine anhydre (35 mL) sous azote, à 0°C. Puis du chlorure de méthanesulfonyle (1.78 mL, 23 mmol) est ajouté goutte à goutte. Après 2h30 d'agitation à température ambiante, le mélange réactionnel est traité avec une solution aqueuse saturée de chlorure d'ammonium (100 mL), puis extrait à l'acétate d'éthyle. Les phases organiques combinées sont ensuite lavées 5 fois avec une solution aqueuse saturée de chlorure d'ammonium, séchées sur sulfate de sodium, filtrées puis concentrées sous vide pour donner le dérivé dimésylé attendu sous forme d'huile jaune.

L'intermédiaire dimésylé est mis en solution dans du diméthylformamide anhydre (45 mL), sous azote, en présence d'azoture de sodium (1.12 g, 17.3 mmol). Le mélange réactionnel est chauffé à 70°C pendant 24 heures. Si nécessaire 1 éq. d'azoture est ajouté pour que la conversion soit complète. Lorsque la réaction est complète, le mélange est traité avec une solution aqueuse 10% de NaH₂PO₄ (100 mL) puis extrait au dichlorométhane. Les phases organiques combinées sont séchées sur sulfate de sodium, filtrées puis concentrées sous vide pour donner l'azoture attendu sous forme d'huile jaune.

L'intermédiaire est mis en réaction, sous azote, dans l'éthanol absolu (17.5 mL). Puis sont ajoutés successivement du di-tert-butyl dicarbonate (1.38 g, 6.34 mmol), du triéthylsilane (1.38 mL, 8.64 mmol) et de l'hydroxyde de palladium sur charbon 10% Degussa (52 mg). Après une nuit à température ambiante, le mélange réactionnel est filtré puis concentré pour donner une huile jaune brute. Ce brut est purifié par chromatographie sur colonne de silice (éluant gradient CH₂Cl₂/MeOH 100/0 à 95/5 par 1%) pour conduire au composé attendu (1.36 g, 3.40 mmol, 34%) sous forme de solide blanc.
MS (ES(+)) : m/z [M+H]⁺ = 401
¹H RMN (400MHz, MeOH-*d₄*) : δ(ppm) - 1.51 (s, 9H, C(CH₃)₃), 3.21-3.59 (m, 4H, N-CH₂-CH-N et N-CH-CH₂-NHBoc), 4.36 (m, 1H, N-CH-CH₂-OH), 4.46 (m, 1H, N-CH₂-CH-N), 4.99 (AB, 2H, CH₂-Ph), 7.41-7.52 (m, 5H, Ph), 7.63 (s, 1H, H pyrazole).

### Stade C :

### trans [[4,8-dihydro-1-tert-butoxycarbamate-6-oxo-5-(phényl-méthoxy)-4,7-méthano-7H-pyrazolo[3,4-e] [1,3] diazépin-8-yl]méthyl]-carbarnate de 1,1-diméthyle

Le composé obtenu au stade B de l'exemple 2 (104 mg, 0.26 mmol) est mis en solution dans du dichlorométhane anhydre (2.5 mL) puis du di-*tert*-butyl dicarbonate (114 mg, 0.52 mmol) et de la diméthylaminopyridine (32 mg, 0.26 mmol) sont ajoutés au mélange. Après 1 nuit d'agitation à température ambiante, le mélange réactionnel est traité avec de l'eau. Les phases sont séparées puis la phase organique est lavée avec une solution aqueuse saturée de chlorure de sodium, séchée sur sulfate de sodium, filtrée puis concentrée sous vide. Le brut ainsi obtenu est purifié par chromatographie sur silice (éluant : CH₂Cl₂/AcOEt 90/10) pour donner le produit attendu (76 mg, 0.15 mmol, 59%).
MS (ES(+)) : m/z [M+H]⁺ = 500

### Stade D :

### sel de pyridinium du trans [[4,8-dihydro-1-tert-butoxycarbamate-6-oxo-5-(sulfooxy)-4,7-méthano-7H-pyrazolo[3,4-e] [1,3] diazépin-8-yl]méthyl]-carbamate de 1,1-diméthyle

Le composé obtenu au stade C de l'exemple 2 (76 mg, 0.15 mmol) est mis en solution, sous azote, dans un mélange diméthylformamide/CH₂Cl₂ 1/3 anhydre (0.87 mL). Du palladium 10% sur charbon à 50% en eau (49 mg) est ajouté. Après trois purges vide/azote, le mélange réactionnel est placé sous atmosphère d'hydrogène jusqu'à disparition du produit de départ en HPLC. Le mélange est alors concentré sous vide puis co-évaporé trois fois avec du dichlorométhane anhydre, enfin séché sous cloche à vide en présence de P₂O₅ pendant 2h.

Le dérivé débenzylé est repris dans de la pyridine anhydre (0.43 mL), sous azote, en présence du complexe pyridine/sulfure de trioxyde (48 mg, 0.30 mmol). Le mélange réactionnel est agité à température ambiante jusqu'à conversion complète en HPLC, puis évaporé à sec après traitement par ajout d'eau. Le brut ainsi obtenu est purifié par chromatographie sur silice (éluant CH₂Cl₂/MeOH 90/10) pour donner le composé attendu (47 mg, 0.083 mmol, 55%).
MS (ES(-)) : m/z [M-2*BOC-H]⁻ = 388
¹H RMN (400MHz, MeOH-*d₄*) : δ(ppm) = 1.52 (s, 18H ,2x C(CH₃)₃), 3.50 (m, 4H, N-CH₂-CH-N et CH₂-NHBoc), 4.62 (m, 1H, CH-CH₂-NHBoc), 4.85 (d, 1H, N-CH₂-CH-N), 7.72 (s, 1H, H pyrazole).

### Stade E :

### sel de sodium et de trifluoroacétate de la trans [[8-(amino-méthyl)-4,8-dihydro-6-oxo-5-(sulfooxy)-4,7-méthano-7H-pyrazolo[3,4-e][1,3]diazépin-6(5H)-one

Une suspension de 6g de résine DOWEX 50WX8 dans une solution de soude 2N (30 mL) est agitée à température ambiante pendant 1h, puis versée sur une colonne à chromatographier. Après rinçage avec H₂O jusqu'à pH neutre, la colonne est conditionnée avec un mélange THF/H₂O 10/90. Le dérivé obtenu au stade D de l'exemple 2 (47 mg, 0.08 mmol) est dissout dans un minimum de méthanol puis déposé sur la colonne. Après élution avec un mélange THF/H₂O 10/90, les fractions contenant le produit attendu sont rassemblées, congelées, puis lyophilisées pour conduire au sel de sodium attendu.

Le sel de sodium est repris dans le dichlorométhane anhydre (1.04 mL) sous azote puis refroidit à 0°C. Une solution d'acide trifluoroacétique/dichlorométhane anhydre 1/1 (2.04 mL) est ajouté goutte à goutte. Le mélange réactionnel est ensuite agité à température ambiante pendant 45 min. Après évaporation à sec puis co-évaporation avec du dichlorométhane anhydre, le composé est repris dans l'eau (~2 mL) puis congelé et lyophilisé pour donner le sel attendu (16 mg, 0.030 mmol, 36%) sous forme de poudre jaune pâle.
MS (ES(-)) : m/z [M-H]⁻ = 288
¹H RMN (400MHz, MeOH-*d₄*) : δ(ppm) - 3.37-3.69 (m, 4H, N-CH₂-CH-N et CH-CH₂-NH₂), 4.81 (dd, 1H, CH-CH₂-NH₂), 4.98 (d, 1H, N-CH₂-CH-N), 7.79 (s, 1H, H pyrazole).

### Exemple 3 Sel de sodium et de trifluoroacétate de la trans [[8-(méthylaminométhyl)-4,8-dihydro-1-méthyl-6-oxo-5-(sulfooxy)-4,7-méthano-7H-pyrazolo[3,4-e] [1,3] diazépin-6(5H)-one

### Stade A :

### Iodure du trans [[[4,8-dihydro-1-méthyl-6-oxo-5-(phénylméthoxy)-4,7-méthano-7H-pyrazolo[3,4-e] [1,3] diazépin-8-yl]méthyl]-méthylamino]triméthylphosphonium

Une solution molaire de triméthylphosphine (1.5 mL, 1.5 mmol) est ajoutée goutte à goutte à une solution du dérivé obtenu au stade E de l'exemple 1 (0.5 g, 1.25 mmol) en solution dans le tétrahydrofurane (15 mL) à température ambiante sous azote et sous agitation. Après 2h d'agitation, l'iodure de méthane (0.21 g, 3.75 mmol) est ajouté au milieu réactionnel. Il se forme rapidement un précipité jaune clair. Après une nuit d'agitation à température ambiante, le milieu réactionnel est concentré sous pression réduite. Le produit brut est trituré dans dichlorométhane. Le précipité est filtré pour donner le produit (0.42 g, 1.04 mmol, 84%) attendu sous forme de sel d'iode de couleur jaunâtre.
MS (ES (+)) : m/z [M+H]⁺ = 402
¹H NMR (400MHz, CDCl₃) sous forme de 2 conformères : δ (ppm) = 2.04 (s, 3H, CH₃P), 2.32 (s, 3H, CH₃P), 2.35 (s, 3H, CH₃P), 3.03 (s. 3H, P-NCH₃(A)-CH₂), 3.05 (s, 3H, P-NCH₃(B)-CH₂), 3.37 (m, 1H, N-CH₂-CH-N ou CH-CH₂-N(CH₃)P), 3.44 (m, 1H, N-CH₂-CH-N ou CH-CH₂-N(CH₃)P), 3.69 (m, 1H, N-CH₂-CH-N ou CH-CH₂-N(CH₃)P), 3.82 (s. 3H, CH₃), 3.88 (m, 1H, N-CH₂-CH-N ou CH-CH₂-N(CH3)P), 4.05 (d, 1H, N-CH₂-CH-N), 4.59 (d, 1H, CH-CH₂-N(CH₃)P), 4.88 (d, 1H, N-O-CH2-Ph), 5.00 (d, 1H, N-O-CH₂-Ph), 7.35 (s, 1H, H pyrazole), 7.37-7.45 (massif, 5H, Ph)

### Stade B :

### trans 8-(méthylaminométhyl)-4,8-dihydro-1-méthyl-5-(phénylméthoxy)-4,7-méthano-7H-pyrazolo[3,4-e] [1,3] diazépin-6(5H)-one

A une solution aqueuse de carbonate de sodium (2.5N, 9 mL) est ajouté le dérivé obtenu au stade A de l'exemple 3 (0.42 g, 1.04 mmol). Le milieu réactionnel est agité à 55°C pendant 3h30. Après refroidissement à température ambiante, le milieu réactionnel est saturé de chlorure de sodium en présence d'acétate d'éthyle (25 mL). La phase aqueuse est extraite avec de l'acétate d'éthyle (3x25 mL). La phase organique est séchée sur sulfate de magnésium puis concentrée sous pression réduite pour délivrer une huile jaune (0.26 g). Le brut réactionnel est purifié par chromatographie sur colonne de silice (éluant dichlorométhane 100% puis gradient avec du méthanol de 2% à 10%) pour donner le dérivé attendu (0.084 g, 0.256 mmol, 26%).
MS (ES (+)) : m/z [M+H]⁺ - 328
¹H NMR (400MHz, CDCl₃) : δ (ppm) = 2.97-3.00 (dd, 1H, N-CH₂-CH-N), 3.00 (CH-CH₂-NCH₃), 3.15 (dd, 1H, CH-CH₂-NCH₃), 3.9 (dd, 1H, N-CH₂-CH-N), 3.75 (s, 3H, CH₃), 3.98 (d, 1H, CH-CH₂-N(CH₃)Boc), 4.72 (dd, 1H, N-CH₂-CH-N), 4.90 (d, 1H, N-O-CH₂-Ph), 5.03 (d, 1H, N-O-CH₂-Ph), 7.30 (s, 1H, H pyrazole), 7.34-7.44 (massif, 5H, Ph)

### Stade C :

### trans [[4,8-dihydro-1-méthyl-6-oxo-5-(phénylméthoxy)-4,7-méthano-7H-pyrazolo[3,4-e] [1,3] diazépin-8-yl]méthyl]-méthyl-carbamate de 1,1-diméthyléthyle

Le dérivé obtenu au stade B de l'exemple 3 (80 mg, 0.244 mmol) est mis en solution dans le dichlorométhane (1 mL) puis à température ambiante est additionné successivement de la triéthyle amine (60 µL, 0.488 mmol) et du di-tert-butyldicarbonate (106 mg, 0.488 mmol). Après 4h d'agitation à température ambiante, une solution saturée de chlorure de sodium (5 mL) est additionnée au milieu réactionnel. La phase aqueuse est extraite par le dichlorométhane (3x20 mL). La phase organique est séchée sur sulfate de magnésium puis concentrée sous pression réduite pour donner une poudre blanche amorphe (157 mg). Le brut réactionnel est chromatographié sur colonne de silice (éluant dichlorométhane 100% puis gradient avec de l'acétate d'éthyle de 20% à 30%) pour donner le dérivé attendu (0.068 g, 0.159 mmol, 60%).
MS (ES (+)) : m/z [M+H] ⁺=428
¹H NMR (400MHz, CDCl₃) : δ (ppm) = 1.59 (s, 9H, C(CH₃)₃), 3.05 (s, 3H, CH₃NBoc-CH₂), 3.10 (m, 3H, N-CH₂-CH-N, CH-CH₂-NBoc), 3.75 (m, 1H, N-CH₂-CH-N), 3.85 (s, 3H, CH₃), 3.99 (s, 1H, N-CH₂-CH-N), 4.75 (m, 1H, CH-CH₂-N(CH₃)Boc), 4.90 (d, 1H, N-O-CH₂-Ph), 5.02 (d, 1H, N-O-CH2-Ph), 7.37 (s, 1H, H pyrazole), 7.40-7.46 (massif, 5H, Ph)

### Stade D :

### Sel de pyridinium du trans [[4,8-dihydro-1-méthyl-6-oxo-5-(sulfooxy)-4,7-méthano-7H-pyrazolo[3,4-e] [1,3] diazépin-8-yl]méthyl]-méthyl-carbamate de 1,1-diméthyléthyle

En procédant comme indiqué au stade G de l'exemple 1, le composé obtenu au stade C de l'exemple 3 (0.068 g, 0.159 mmol) dans le méthanol (5 mL), en présence de palladium 10% sur charbon (25 mg) conduisent au produit débenzylé.
MS (ES (+)) : m/z [M+H]⁺ = 337
L'intermédiaire débenzylé, la pyridine (1 mL), le complexe pyridine/sulfure de trioxyde (50 mg, 0.318 mmol) conduisent au sel attendu (0.045 g, 0.090 mmol, 100%).
MS (ES (-)) : m/z [M-H]⁻ = 416
¹H NMR (400 MHz, MeOH-*d₄*) sous forme de 2 conformères : δ (ppm) = 1.53 (s, 9H, C(CH₃)₃, 3.09 (s, 3H, CH₃(A)NHBoc), 3.10 (s, 3H, CH₃(B)NHBoc), 3.37 (m, 1H, BocN(CH₃)-CH₂-CH ou N-CH₂-CH-N), 3.58 (m, 1H, BocN(CH₃)-CH₂-CH ou N-CH₂-CH-N), 3.75 (s, 3H, CH₃), 3.84 (m, 1H, BocN(CH₃)-CH₂-CH ou N-CH₂-CH-N), 3.90(m, 1H, BocN(CH₃)-CH₂-CH ou N-CH₂-CH-N), 4.90 (m, 2H, N-CH-CH₂-N, N-CH₂-CH-N + signal H₂O), 7.54 (s, 1H, H pyrazole), 8.16 (dd, 2H, pyridine), 8.70 (dd, 2H, pyridine), 8.94 (d, 1H, pyridine)

### Stade E :

### Sel de sodium de la trans [[4,8-dihydro-2-méthyl-6-oxo-5-(sulfooxy)-4,7-méthano-7H-pyrazolo[3,4-e] [1,3] diazépin-8-yl]méthyl]-méthyl-carbamate de 1,1-diméthyléthyle

En procédant comme indiqué au stade H de l'exemple 1, le sel obtenu au stade D de l'exemple 3 (0.045 g, 0.090 mmol), la résine DOWEX 5OWX8 (30 g) et la soude 2N (150 mL) conduisent au sel de sodium attendu (0.039 g, 0.090 mmol, 100%).
MS (ES (-)) : m/z [M-H]⁻ = 416
¹H NMR (400 MHz, MeOH-*d₄*) sous forme de 2 conformères : δ (ppm) =1.56 (s, 9H, C(CH₃)₃), 3.09(s, 3H, CH₃(A)NHBoc), 3.10 (s, 3H, CH₃(B)NHBoc), 3.37 (m, 1H, BocN(CH₃)-CH₂-CH ou N-CH₂-CH-N), 3.64 (m, 1H, BocN(CH₃)-CH₂-CH ou N-CH₂-CH-N), 3.75 (s, 3H, CH₃), 3.84 (m, 1H, BocN(CH₃)-CH₂-CH ou N-CH₂-CH-N), 3.93 (m, 1H, BocN(CH₃)-CH₂-CH ou N-CH₂-CH-N), 4.90 (m, 2H, N-CH-CH₂-N, N-CH₂-CH-N + signal H₂O), 7.55 (s, 1H, H pyrazole).

### Stade F :

### Sel de sodium et de trifluoroacétate de la trans 8-(méthylaminométhyl)-4,8-dihydro-1-méthyl-6-oxo-5-(sulfooxy)-4,7-méthano-7H-pyrazolo[3,4-e] [1,3] diazépin-6(5H)-one

En procédant comme indiqué au stade I de l'exemple 1, le sel de sodium obtenu au stade E de l'exemple 3 (0.039 g, 0.088 mmol), le dichlorométhane (5 mL) et un mélange d'acide trifluoroacétique/dichlorométhane anhydre 1/1 (4 mL) conduisent au produit attendu (39 mg, 0.08 mmol, 100%).
MS (ES (-)) : m/z [M-H]⁻ = 315
¹H NMR (400 MHz, DMSO-*d*₆) _{:} δ (ppm) = 2.76 (s, 3H, CH3NH⁺₂-CH₂), 3.30-3.50 (m, 4H, N-CH₂-CH-N, NH⁺₂-CH₂-CH), 3.75 (s, 3H, CH₃), 4.74 (m, 1H, N-CH₂-CH-N), 4.82 (d, 1H, CH-CH₂-NH⁺₂CH₃), 7.43 (s, 1H, H pyrazole), 8.67 (m, 2H, NH₃⁺)

### Exemple 4: Composition pharmaceutique

On a préparé une composition pour injection renfermant:
- Composé de l'exemple 1: 500 mg
- Excipient aqueux stérile: q.s.p. 5 cm³

### ETUDE PHARMACOLOGIQUE DES PRODUITS DE L'INVENTION

### Activité in vitro, méthode des dilutions en milieu liquide :

On prépare une série de tubes dans lesquels on répartit la même quantité de milieu nutritif stérile, on distribue dans chaque tube des quantités croissantes du produit à étudier puis chaque tube est ensemencé avec une souche bactérienne. Après incubation de 24 heures à l'étuve à 37°C, l'inhibition de la croissance est appréciée par transillumination, ce qui permet de déterminer les concentrations minimales inhibitrices (C.M.I.) exprimées en µg/ml.

On effectue ainsi des tests avec les produits des exemples 1 et 2 en comparaison avec les produits des exemples 18 et 19 de la demande WO 02/100860. Les produits des exemples 1 et 2 se révèlent très actifs sur *Pseudomonas aeruginosa,* ce qui n'est pas du tout le cas des produits de comparaison.

| **Activité sur Pseudomonas aeruginosa** (comparaison avec les autres exemples) (MIC (µg/mL) 24 h) | Pdt Ex 1 | Pdt Ex 2 | Pyrazole amide (Ex 19 Demande de brevet WO 02/1000860) | Pyrazole ester (Ex18 demande de brevet WO 02/1000860) |
|---|---|---|---|---|
| 1771 souche type sauvage | 0,12 | 0,25 | >32 | >32 |

| **Souches de Pseudomonas aeruginosa exprimant des mécanismes de résistance** (MIC (µg/mL) 24 h) | Pdt Ex 1 | Pdt Ex 2 | Imipenem | Ceftazidime |
|---|---|---|---|---|
| 1771 type sauvage | 0,12 | 0,25 | 1 | 1 |
| PAO1 type sauvage | 1 | 1-1 | 1 | 2 |
| Résistantes via β-[8 souches] | 1-2 | 0,25-1 | 2-32 | 2-32 |
| Résistantes via pompes à efflux [11/5 souches] | 0,5- 4 / 0,25-0,5 | - / 0,5-1 | 0,25- 4 | <= 0,03-8 |
| Résistantes via mutation de porine [1 souche] | 2 | - | 16 | 2 |

## Revendications

1. Les composés de formule générale (I) : 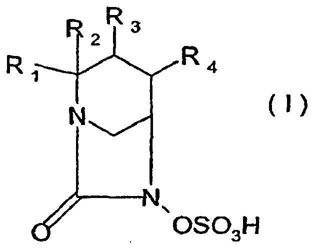 dans laquelle Rₗ₁ représente un radical (CH₂)ₙ-NH₂ ou (CH₂)ₙ-NHR, R étant un alkyle (C₁-C₆) et n étant égal à 1 ou 2 ;
R₂ représente un atome d'hydrogène ;
R₃ et R₄ forment ensemble un hétérocycle azoté à caractère aromatique à 5 sommets renfermant 1, 2 ou 3 atomes d'azote éventuellement substitué par un ou plusieurs groupements R', R' étant choisi dans le groupe constitué par un atome d'hydrogène, les radicaux alkyle renfermant de 1 à 6 atomes de carbone ;
sous forme libre, de zwitterions et sous forme de sels avec les bases et les acides minéraux ou organiques pharmaceutiquement acceptables.

2. Les composés selon la revendication 1, **caractérisés en ce que** R₃ et R₄ forment ensemble un radical pyrazolyle ou triazolyle éventuellement substitué.

3. Les composés selon la revendication 1 ou 2, **caractérisés en ce que** R₁ est choisi dans le groupe constitué par les groupements (CH₂)ₙ-NH₂ et (CH₂)ₙ-NHCH₃, n étant tel que défini à la revendication 1, l'hétérocycle formé par R₃ et R₄ est substitué par un radical alkyle (C₁-C₆).

4. Les composés selon l'une quelconque des revendications 1 à 3, **caractérisé en ce que** R₁ représente un radical (CH₂)ₙ-NH₂ ou (CH₂)ₙ-NHCH₃, n étant tel que défini à la revendication 1 et R₃ et R₄ forment ensemble un cycle pyrazolyle substitué par un radical alkyle (C₁-C₆).

5. - la trans 8-(aminométhyl)-4,8-dihydro-1-méthyl-6-oxo-5-(sulfooxy)- 4,7-méthano-7H-pyrazolo[3,4-e] [1,3]diazépin-6(5H)-one,
- la trans 8-(aminométhyl)-4,8-dihydro-6-oxo-5-(sulfooxy)-4,7-méthano-7H-pyrazolo[3,4-e] [1,3]diazépin-6(5H)-one,
- la trans 8-(méthylaminométhyl)-4,8-dihydro-6-oxo-5-(sulfooxy)- 4,7-méthano-7H-pyrazolo[3,4-e] [1,3]diazépin-6(5H)-one,
sous forme libre, de zwittterion et de sels avec les bases et les acides minéraux ou organiques pharmaceutiquement acceptables.

6. Procédé pour la préparation des composés de formule (I), **caractérisé en ce qu'**il comporte :
a) une étape au cours de laquelle on fait réagir, avec un agent de carbonylation, le cas échéant en présence d'une base, un composé de formule (II) : 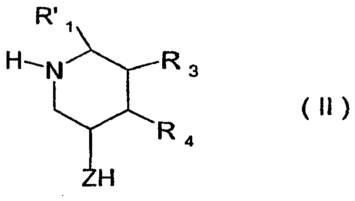 dans laquelle:
R'₁ représente un radical CN, COOH protégé, COOR ou (CH₂)ₙR'₅.
R'₅ est un radical OH protégé, CN, NH₂ ou NHR protégé, CO2H protégé, CO2R,
n, R, R₃ et R₉ sont tels que définis ci-dessus, le substituant aminoalkyle éventuellement présent sur l'hétérocycle formé par R₃ et R₄ étant alors le cas échéant protégé,
ZH représente un groupement -NHOH protégé,
en vue d'obtenir un composé intermédiaire de formule (III) : 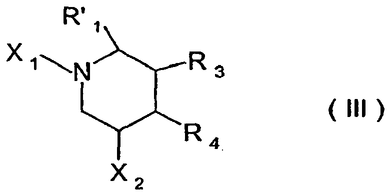 dans laquelle :
R'₁, R₃ et R₄ ont les mêmes significations que ci-dessus et soit X₁ est un atome d'hydrogène ou un groupe protecteur et X₂ représente un groupement -Z-CO-X₃, X₃ représentant le reste de l'agent de carbonylation, soit X₂ est un groupement -ZH et X₁ représente un groupement CO-X₃, X₃ étant défini comme ci-dessus ;
b) une étape au cours de laquelle on cyclise l'intermédiaire obtenu précédemment, en présence d'une base ;
et **en ce que** :
c) le cas échéant, l'étape a) est précédée et/ou l'étape b) est suivie de l'une ou de plusieurs des réactions suivantes, dans un ordre approprié :
- protection des fonctions réactives,
- déprotection des fonctions réactives,
- estérification
- saponification,
- sulfatation,
- réduction d'esters,
- alkylation,
- carbamoylation,
- formation d'un groupe azido,
- réduction d'un azido en amine,
- salification,
- échange d'ions,
- dédoublement ou séparation de diastéréoisomères.

7. Procédé selon la revendication 6, **caractérisé en ce que** l'agent de carbonylation est choisi dans le groupe constitué par le phosgène, le diphosgène, le triphosgène, les chloro-formiates d'aryle, d'aralkyle, d'alkyle et d'alkényle, les dicarbonates d'alkyle, le carbonyl-diimidazole et leurs mélanges.

8. Procédé selon la revendication 6 ou 7, **caractérisé en ce que** la réaction de carbonylation a lieu en présence d'une amine.

9. Procédé selon l'une quelconque des revendications 6 à 8, **caractérisé en ce que** le composé de formule (II) est obtenu par un procédé selon lequel on traite un composé de formule (IV) : 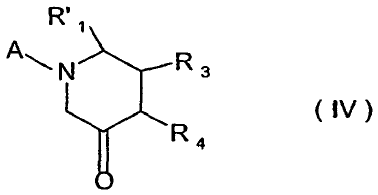 dans laquelle R'1, R₃ et R₄ sont définis comme à la revendication 6, et A représente un atome d'hydrogène ou un groupement protecteur de l'azote, par un agent de réduction, pour obtenir un composé de formule (V) : 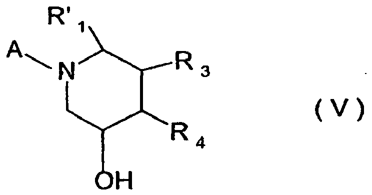 dans laquelle A, R'₁, R₃ et R₄ conservent leur signification précitée, dans lequel, le cas échéant, l'on remplace le groupement OH par un groupe partant, pour obtenir un composé de formule (VI) : 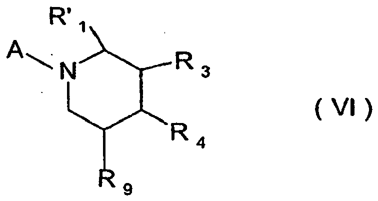 dans laquelle A, R'1, R₃ et R₄ conservent leur signification précitée et R₉ représente un groupe partant, que l'on traite par un composé de formule Z₁H₂ dans laquelle Z₁ représente un groupement -HN-OH protégé puis, le cas échéant, par un agent de déprotection de l'atome d'azote approprié.

10. Procédé selon l'une quelconque des revendications 6 à 8, **caractérisé en ce que** le composé de formule (II) est obtenu par un procédé selon lequel on traite un composé de formule (IV) telle que définie précédemment, par l'hydroxylamine protégée au niveau de l'hydroxy, pour obtenir un composé de formule (VII) : 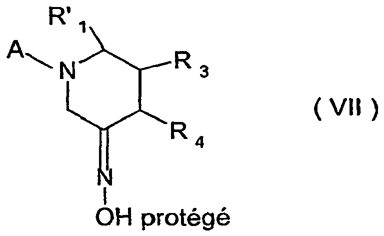 dans laquelle A, R'₁, R₃ et R₄ sont définis comme à la revendication 9, que l'on fait réagir avec un agent de réduction pour obtenir un composé de formule (VIII) : 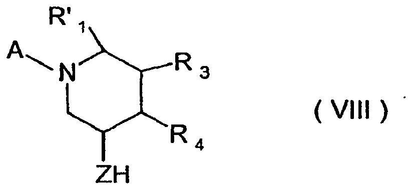 dans laquelle A, R'₁, R₃, R₄, n" et ZH sont définis comme précédemment, que l'on traite, le cas échéant, par un agent de déprotection de l'atome d'azote approprié.

11. Médicaments comprenant les produits tels que définis à l'une quelconque des revendications 1 à 4, ainsi que leurs sels avec des acides et des bases pharmaceutiquement acceptables.

12. Médicaments comprenant les produits tels que définis à la revendication 5.

13. Compositions pharmaceutiques contenant, à titre de principe actif, au moins un médicament selon l'une des revendications 11 ou 12.

## Patentansprüche

1. Verbindungen der allgemeinen Formel (I): 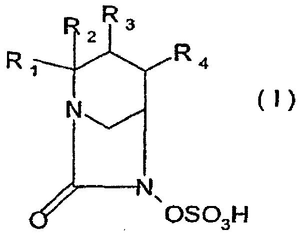
worin R₁ für einen (CH₂)ₙ-NH₂- oder (CH₂)ₙ-NHR-Rest steht, wobei R einen (C₁-C₆)-Alkylrest bedeutet und n gleich 1 oder 2 ist;
R₂ für ein Wasserstoffatom steht;
R₃ und R₄ zusammen einen 5-gliedrigen StickstoffHeterocyclus mit aromatischem Charakter bilden, der 1, 2 oder 3 Stickstoffatome enthält und gegebenenfalls durch eine oder mehrere Gruppen R' substituiert ist, wobei R' aus der Gruppe bestehend aus einem Wasserstoffatom und Alkylresten mit 1 bis 6 Kohlenstoffatomen ausgewählt ist;
in freier Form und in Form von Zwitterionen und in Form von Salzen mit pharmazeutisch annehmbaren anorganischen oder organischen Basen und Säuren.

2. Verbindungen nach Anspruch 1, **dadurch gekennzeichnet, dass** R₃ und R₄ zusammen einen gegebenenfalls substituierten Pyrazolyl- oder Triazolylrest bilden.

3. Verbindungen nach Anspruch 1 oder 2, **dadurch gekennzeichnet, dass** R₁ aus der Gruppe bestehend aus (CH₂)ₙ-NH₂- und (CH₂)ₙ-NHCH₃-Gruppen ausgewählt ist, wobei n wie in Anspruch 1 definiert ist, und der durch R₃ und R₄ gebildete Heterocyclus durch einen (C₁-C₆)-Alkylrest substituiert ist.

4. Verbindungen nach einem der Ansprüche 1 bis 3, **dadurch gekennzeichnet, dass** R₁ für einen (CH₂)ₙ-NH₂- oder (CH₂)ₙ-NHCH₃-Rest steht, wobei n wie in Anspruch 1 definiert ist, und R₃ und R₄ zusammen einen durch einen (C₁-C₆)-Alkylrest substituierten Pyrazolylrest bilden.

5. - trans-8-(Aminomethyl)-4,8-dihydro-1-methyl-6-oxo-5-(sulfooxy)-4,7-methano-7H-pyrazolo[3,4-e][1,3]diazepin-6(5H)-on,
- trans-8-(Aminomethyl)-4,8-dihydro-6-oxo-5-(sulfooxy)-4,7-methano-7H-pyrazolo[3,4-e]-[1,3]diazepin-6(5H)-on,
- trans-8-(Methylaminomethyl)-4,8-dihydro-6-oxo-5-(sulfooxy)-4,7-methano-7H-pyrazolo[3,4-e]-[1,3]diazepin-6(5H)-on,
in freier Form und in Form von Zwitterionen und in Form von Salzen mit pharmazeutisch annehmbaren anorganischen oder organischen Basen und Säuren.

6. Verfahren zur Herstellung der Verbindungen der Formel (I), **dadurch gekennzeichnet, dass** es Folgendes umfasst:
a) einen Schritt, bei dem man eine Verbindung der Formel (II) : 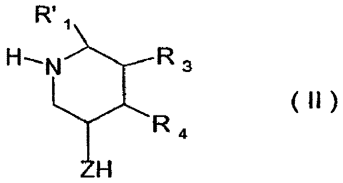 worin:
R'₁ für einen CN-, geschützten COOH-, COOR- oder (CH₂)ₙR'₅-Rest steht,
R'₅ für einen geschützten OH-, CN-, NH₂- oder geschützten NHR-, geschützten CO₂H- oder CO₂R-Rest steht,
wobei n, R, R₃ und R₄ wie oben definiert sind, wobei der gegebenenfalls an dem durch R₃ und R₄ gebildeten Heterocyclus vorliegende Aminoalkylsubstituent gegebenenfalls geschützt ist,
ZH für eine geschützte -NHOH-Gruppe steht, gegebenenfalls in Gegenwart einer Base mit einem Carbonylierungsmittel zu einer Zwischenverbindung der Formel (III): 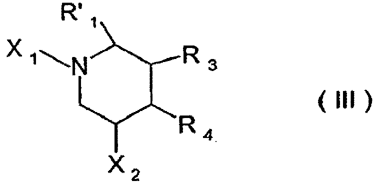 worin:
R'₁, R₃ und R₄ die gleichen Bedeutungen wie oben besitzen und entweder X₁ für ein Wasserstoffatom oder eine Schutzgruppe steht und X₂ für eine -Z- CO-X₃-Gruppe steht, wobei X₃ für den Rest des Carbonylierungsmittels steht, oder X₂ für eine -ZH-Gruppe steht und X₁ für eine CO-X₃-Gruppe steht, wobei X₃ wie oben definiert ist, umsetzt;
b) einen Schritt, bei dem man das oben erhaltene Zwischenprodukt in Gegenwart einer Base cyclisiert;
und dass:
c) gegebenenfalls eine oder mehrere der folgenden Reaktionen in einer geeigneten Reihenfolge dem Schritt a) vorgeschaltet und/oder dem Schritt b) nachgeschaltet werden:
- Schützung reaktiver Funktionen,
- Entschützung reaktiver Funktionen,
- Veresterung,
- Verseifung,
- Sulfatierung,
- Reduktion von Estern,
- Alkylierung,
- Carbamoylierung,
- Bildung einer Azidogruppe,
- Reduktion von Azido zu Amin,
- Versalzung,
- Ionenaustausch,
- Racematspaltung oder Trennung von Diastereoisomeren.

7. Verfahren nach Anspruch 6, **dadurch gekennzeichnet, dass** man das Carbonylierungsmittel aus der Gruppe bestehend aus Phosgen, Diphosgen, Triphosgen, chlorameisensäurearylestern, -aralkylestern, -alkylestern und -alkenylestern, Alkyldicarbonaten, Carbonyldiimidazol und Mischungen davon auswählt.

8. Verfahren nach Anspruch 6 oder 7, **dadurch gekennzeichnet, dass** die Carbonylierungsreaktion in Gegenwart eines Amins stattfindet.

9. Verfahren nach einem der Ansprüche 6 bis 8, **dadurch gekennzeichnet, dass** die Verbindung der Formel (II) durch ein Verfahren erhalten wird, gemäß dem man eine Verbindung der Formel (IV): 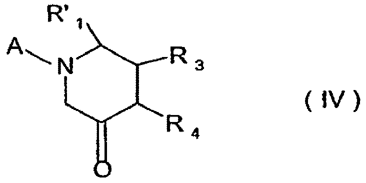 worin R'_{1,} R₃ und R₄ wie in Anspruch 6 definiert sind und A für ein Wasserstoffatom oder eine Stickstoff-Schutzgruppe steht, behandelt, wobei man eine Verbindung der Formel (V): 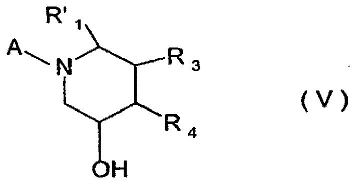 worin A, R'₁, R₃ und R₄ ihre oben angegebene Bedeutung behalten, erhält, wobei man gegebenenfalls die OH-Gruppe durch eine Abgangsgruppe ersetzt, wobei man eine Verbindung der Formel (VI) : 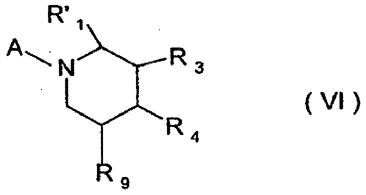 worin A, R'₁, R₃ und R₄ ihre oben angegebene Bedeutung behalten und R₉ für eine Abgangsgruppe steht, erhält, welche man mit einer Verbindung der Formel Z₁H₂, worin Z₁ für eine geschützte -HN-OH-Gruppe steht, und dann gegebenenfalls mit einem geeigneten Entschützungsmittel für das Stickstoffatom behandelt.

10. Verfahren nach einem der Ansprüche 6 bis 8, **dadurch gekennzeichnet, dass** die Verbindung der Formel (II) durch ein Verfahren erhalten wird, gemäß dem man eine Verbindung der Formel (IV) gemäß obiger Definition mit einem an der OH-Gruppe geschützten Hydroxylamin behandelt, wobei man eine Verbindung der Formel (VII): 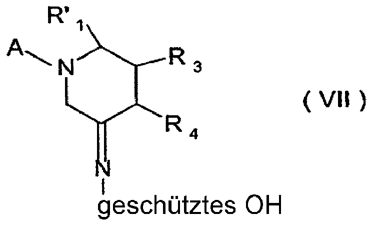 worin A, R'₁, R₃ und R₄ wie in Anspruch 9 definiert sind, erhält, welche man mit einem Reduktionsmittel zu einer Verbindung der Formel (VIII): 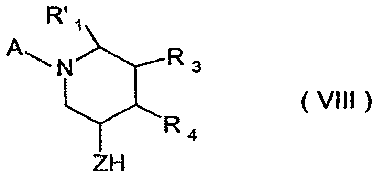 worin A, R'₁, R₃, R₄, n" und ZH wie oben definiert sind, umsetzt, welche man gegebenenfalls mit einem geeigneten Entschützungsmittel für das Stickstoffatom behandelt.

11. Medikamente, umfassend die Produkte gemäß einem der Ansprüche 1 bis 4 sowie Salze davon mit pharmazeutisch annehmbaren Säuren und Basen.

12. Medikamente, umfassend die Produkte gemäß Anspruch 5.

13. Pharmazeutische Zusammensetzungen, die als Wirkstoff mindestens ein Medikament nach einem der Ansprüche 11 oder 12 enthalten.

## Claims

1. Compounds with the general formula (I) 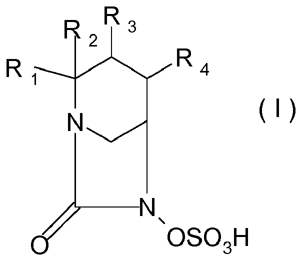
wherein R₁ represents a (CH₂)ₙ-NH₂ or (CH2)ₙ-NHR radical, where R is a (C₁-C₆) alkyl and n is equal to 1 or 2;
R₂ represents a hydrogen atom;
R₃ and R₄ together form an aromatic nitrogenated heterocycle with 5 apexes with 1, 2 or 3 nitrogen atoms optionally substituted by one or several R' groups, R' being selected in the group composed of a hydrogen atom and alkyl radicals with 1 to 6 carbon atoms;
in free form, as zwitterions, and in the form of salts of pharmaceutically acceptable inorganic or organic bases and acids.

2. Compounds according to claim 1, **characterised in that** R₃ and R₄ together form an optionally substituted pyrazolyl or triazolyl radical.

3. Compounds according to claims 1 or 2, **characterised in that** R₁ is selected from the group composed of (CH₂)ₙ-NH₂ and (CH₂)ₙ-NHCH₃ groups, where n is as defined in claim 1, the heterocycle formed by R₃ and R₄ is substituted by a (C₁-C₆) alkyl radical.

4. Compounds according to claims 1 or 3, **characterised in that** R₁ represents a (CH₂)ₙ-NH₂ or (CH₂)n-NHCH₃ radical, where n is as defined in claim 1 and R₃ and R₄ form a pyrazolyl ring substituted by a (C₁-C₆) alkyl radical.

5. - Trans 8-(aminomethyl)-4,8-dihydro-1-methyl-6-oxo-5(sulphooxy)-4,7-methano-7H-pyrazolo[3,4-e] [1,3]diazepin-6(5H)-one,
- trans 8-(aminomethyl)- 4,8-dihydro-6-oxo-5-(sulphooxy)-4,7-methano-7H-pyrazolo[3,4-e] [1,3]diazepin-6(5H)-one,
- trans 8-(methylaminomethyl)- 4,8-dihydro-6-oxo-5-(sulphooxy)-4,7-methano-7H-pyrazolo[3,4-e] [1,3]diazepin-6(5H)-one,
in free form, as zwitterions, and in the form of salts of pharmaceutically acceptable inorganic or organic bases and acids.

6. A method for preparing compounds of formula (I), **characterised in that** it comprises:
a) a step during which a compound of formula (II) is made to react with a carbonylating agent, if necessary in the presence of a base: 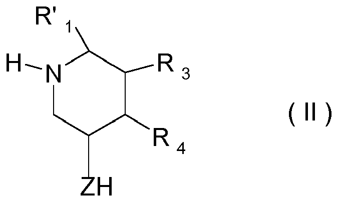 wherein:
R'₁ represents a CN, protected COOH, COOR or (CH₂)ₙR'₅ radical,
R'₅ is a protected OH, CN, NH₂ or protected NHR, protected CO₂H, CO₂R radical,
n, R, R₃ and R₄ are as defined above, the aminoalkyl substituents optionally present on the heterocycle formed by R₃ and R₄ being protected if necessary,
ZH represents a protected -NHOH group,
with the end of obtaining an intermediate compound with the formula (III): 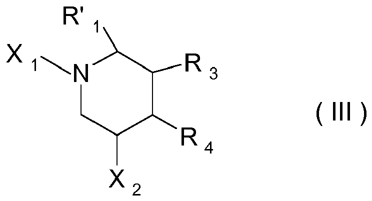 wherein:
R'₁, R₃ and R₄ have the same meanings as above and either X₁ is a hydrogen atom or a protecting group and X₂ represents a -Z-CO-X₃ group, X₃ representing the rest of the carbonylating agent, or X₂ is a -ZH group and X₁ represents a CO-X₃ group, X₃ being defined as above;
b) a step during which the intermediate obtained above is cyclised in the presence of a base;
and **in that**:
c) if necessary, step a) is preceded and/or step b) is followed by one or several of the following reactions, in an appropriate order:
- protection of the reactive functions,
- deprotection of the reactive functions,
- esterification
- saponification,
- sulphatation,
- ester reduction,
- alkylation,
- carbamoylation,
- formation of an azido group,
- reduction of an azido into an amine,
- salification,
- ion exchange,
- dividing or separating the diastereoisomers.

7. A method according to claim 6, **characterised in that** the carbonylation agent is selected from the group composed of phosgene, diphosgene, triphosgene, aryl, aralkyl, alkyl and alkenyl chloro-formiates, alkyl dicarbonates, carbonyldiimidazole and mixtures of these.

8. A method according to claim 6 or 7, **characterised in that** the carbonylation reaction takes place in the presence of an amine.

9. A method according to any of claims 6 to 8, **characterised in that** the compound of formula (II) is obtained by a method wherein a compound of formula (IV) is processed: 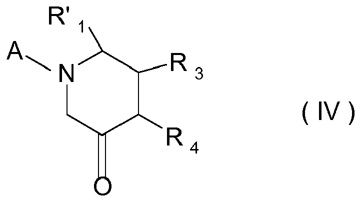 wherein R'₁, R₃ and R₄ are defined as in claim 6, and A represents a hydrogen atom or a group protecting the nitrogen, by a reducing agent, in order to obtain a compound of formula (V) : 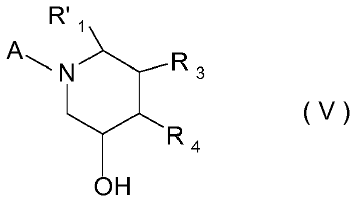 wherein A, R'₁, R₃ and R₄ keep the meanings mentioned above, wherein, if necessary, the OH group is replaced by a leaving group, to obtain a compound of formula (VI): 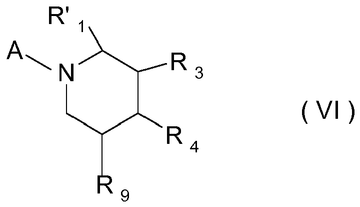 wherein A, R'₁, R₃ and R₄ keep the meanings mentioned above and R₉ represents a leaving group, that is processed with a compound of formula Z₁H₂ wherein Z₁ represents a protected -HN-OH group and then, if necessary, by an appropriate deprotection agent of the nitrogen atom.

10. A method according to any of claims 6 to 8, **characterised in that** the compound of formula (II) is obtained by a method wherein a compound of formula (IV) as defined above, is processed by hydroxylamine protected at the hydroxyl group, to obtain a compound of formula (VII): 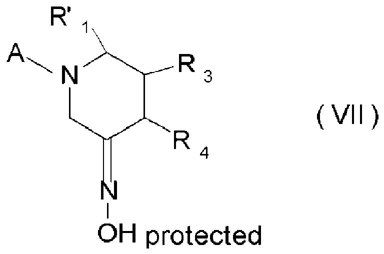 wherein A, R'₁, R₃ and R₄ are defined as in claim 9, which is made to react with a reducing agent in order to obtain a compound of formula (VIII): 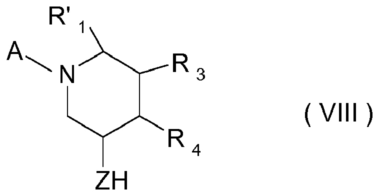 wherein A, R'₁, R₃, R₄, n" and ZH are defined as above, and are processed, if necessary, by an appropriate deprotection agent of the nitrogen atom.

11. Drugs comprising the products as defined in one of claims 1 to 4, and their salts with pharmaceutically acceptable acids and bases.

12. Drugs comprising the products as defined in claim 5.

13. Pharmaceutical compositions containing, as active principle, at least one drug according to one of claims 11 or 12.
